# EUROPÄISCHE PATENTANMELDUNG

(11) **EP 0 902 001 A1**
(43) Veröffentlichungstag der Anmeldung: **17.03.1999**
(21) Anmeldenummer: 98116052.6
(22) Anmeldetag: 26.08.1998
(51) Int. Cl.: C07C 45/51, C07C 49/203, C07C 41/28, C07C 41/08, B01J 23/06

(54) **Verfahren zur Herstellung von ungesättigten Ketonen**

(30) Priorität: 10.09.1997 DE 19739716
(71) Anmelder: BASF AKTIENGESELLSCHAFT, 67056 Ludwigshafen (DE)
(72) Erfinder: Teles, Joaquim Henrique, Dr., 67122 Altrip (DE); Rieber, Norbert, Dr., 68259 Mannheim (DE); Breuer, Klaus, Dr., 67122 Altrip (DE); Demuth, Dirk, Dr., 68161 Mannheim (DE); Hibst, Hartmut, Prof. Dr., 69198 Schriesheim (DE); Käshammer, Stefan, Dr., 67105 Schifferstadt (DE); Etzrodt, Heinz, Dr., 67434 Neustadt (DE); Kaiser, Wulf, Dr., 67098 Bad Dürkheim (DE)

(57) **Zusammenfassung**

Verfahren zur Herstellung von ungesättigten Ketonen der Formeln Ia bzw. Ib

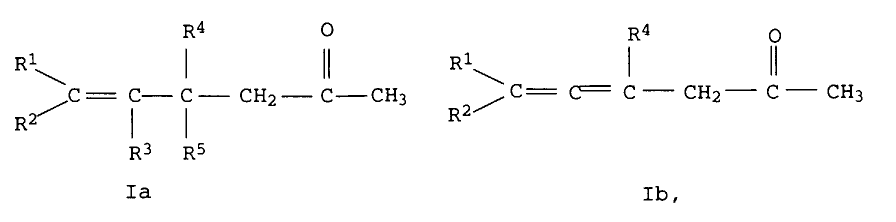

In denen R¹, R², R³, R⁴ und R⁵ Wasserstoff oder gegebenenfalls durch sauerstoffhaltige Gruppen substituierte Alkyl-, Alkenyl-, Cycloalkyl-, Cycloalkenyl-, Aryl- oder Aralkylreste bedeuten, wobei die Reste R¹ und R² auch zusammen einen 5- oder 6-gliedrigen Ring bilden können, bestehend aus der Kombination der folgenden Umsetzungen:
a) Die an sich bekannte Umsetzung von Allylalkoholen der Formel IIa bzw. Propargylalkoholen der Formel IIb 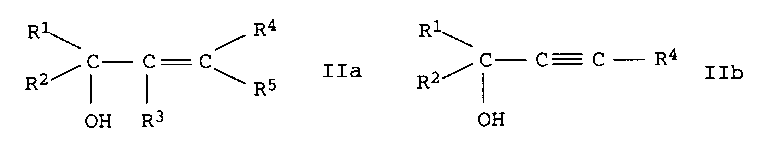 mit Isopropenylethern der Formel III 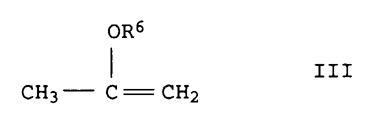 in der R⁶ für einen Alkylrest mit 1 bis 4 C-Atomen steht unter Bildung von Ketalen der Formel IV 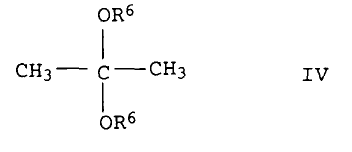 als Nebenprodukt, in der R⁶ die oben genannte Bedeutung hat,
b) Herstellung der Isopropenylether der Formel III durch Umsetzung von Ketalen der Formel der Formel IV mit Propin oder Allen oder deren Mischungen in der Gasphase bei erhöhter Temperatur in Gegenwart eines Zink oder Cadmium zusammen mit Silicium und Sauerstoff enthaltenden Heterogenkatalysators, und
c) Einspeisung des in der Reaktion (a) entstandenen Ketals der Formel IV in die Stufe (b) zur erneuten Herstellung des Isopropenylethers der Formel III.

## Beschreibung

Die Erfindung betrifft die Herstellung von γ,δ-monoungesättigten oder β,γ,δ-diungesättigen Ketonen, die wertvolle Riechstoffe bzw. wertvolle Zwischenprodukte für die Synthese von Naturstoffen sind, durch die Kombination der an sich bekannten Umsetzung von Allylalkoholen bzw. Propargylalkoholen mit Isopropenylethern unter Bildung von Ketalen des Acetons als Nebenprodukt, wobei man die Isopropenylether durch Umsetzung von Ketalen des Acetons mit Propin oder Allen in der Gasphase an heterogenen Katalysatoren herstellt und wobei man das als Nebenprodukt erhaltene Ketal des Acetons in die genannte Isopropenylether-Herstellung einspeist.

Aus DE 1 193 490 sowie aus R. Marbet, G. Saucy, Helv. Chim. Acta (1967), 50, 2091-2095 und 2095-2100 ist ein Verfahren zur Herstellung von γ,δ-ungesättigten Ketonen durch Umsetzung eines Allylalkohols mit Enolethern, insbesondere Isopropenylethern, in Gegenwart eines sauren Katalysators, z.B. Phosphorsaure, bekannt.

Ferner ist aus US 3,029,287 bzw. aus G. Saucy und R. Marbet, Helv. Chim. Acta (1967), 50, 1158 - 1167 die Umsetzung von Propargylalkoholen mit Enolethern in Gegenwart saurer Katalysatoren zu β,γ,δ-diungesättigten Ketonen bekannt.

Bei beiden Reaktionen entsteht aus 2 Mol eingesetztem Enolether ein Mol des entsprechenden Ketals als Nebenprodukt, z.B. aus eingesetztem Isopropenylmethylether, das Acetondimethylketal, gemäß den folgenden Gleichungen

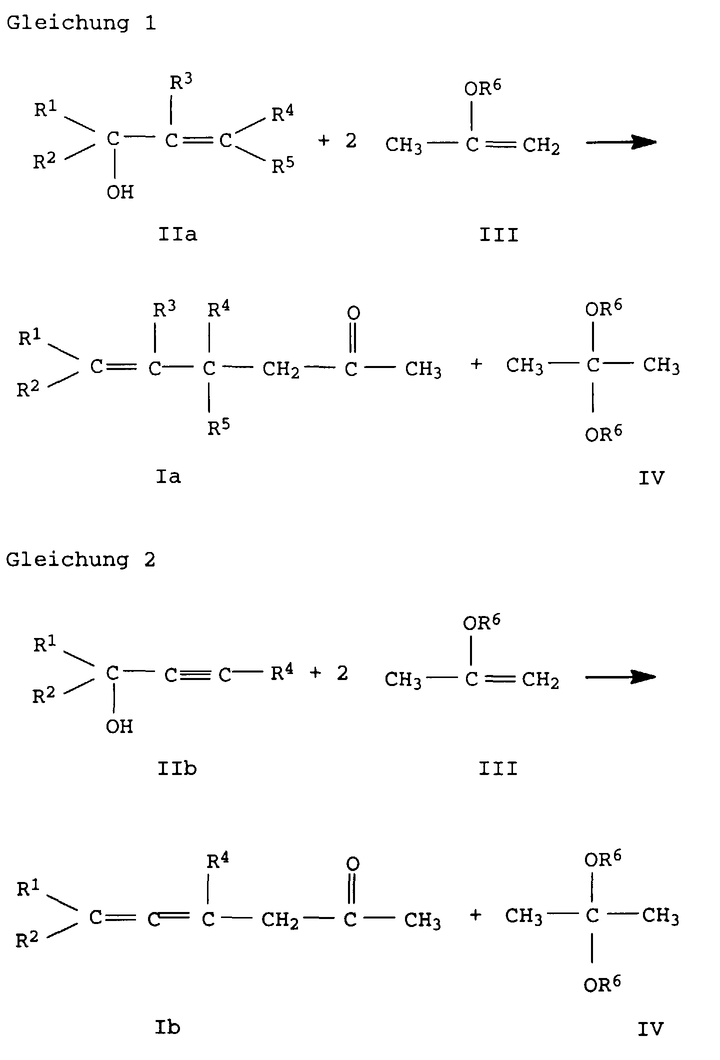

In diesen Gleichungen bedeuten R¹ bis R⁵ Wasserstoff oder gegebenenfalls durch sauerstoffhaltige Gruppen substituierte Alkyl-, Alkenyl-, Cycloalkyl-, Cycloalkenyl-, Aryl- oder Aralkylreste, wobei die Reste R¹ und R² auch zusammen mit dem C-Atom, an das sie gebunden sind, einen 5- oder 6-Ring bilden können und R⁶ einen Alkylrest mit 1 bis 4 C-Atomen.

Das als Nebenprodukt erhaltene Ketal IV muß aus Wirtschaftlichkeitsgründen wieder in den Enolether III überführt werden. Dazu ist bekannt, die Ketale entweder in der Flüssigphase mit sauren Katalysatoren (gemäß EP 703 211) oder in der Gasphase an heterogenen Katalysatoren (gemäß DE 19 544 450) unter Alkoholabspaltung in die entsprechenden Enolether nach der folgenden Reaktionsgleichung zu überführen: 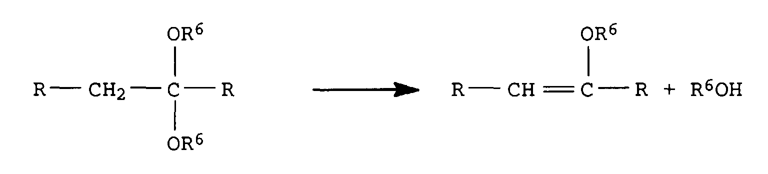 R bedeutet dabei Wasserstoff oder einen Alkylrest und R⁶ hat die oben angegebene Bedeutung.

Die genannten vorbekannten Verfahren erlauben die Herstellung der Enolether zum Teil in guten Ausbeuten, haben jedoch die folgenden Nachteile:

Die Umsetzung in flüssiger Phase gemäß EP 703 211 erfordert die Verwendung einer gelosten Fremdsubstanz, nämlich einer organischen Säure, deren Entfernung aus dem Reaktionsgemisch zusätzlichen Trennaufwand erfordert. Das Verfahren gemäß DE 19544450 hat zwar gegenüber den Verfahren in flüssiger Phase mit homogen gelöstem Katalysator den Vorteil der Reaktion in der Gasphase über einen Heterogenkatalysator, erfordert jedoch ziemlich hohe Temperaturen.

Beiden Verfahren ist gemeinsam, daß pro Mol Ketal ein Mol Alkohol freigesetzt wird, der mit zusätzlichem und z.T. erheblichem Reinigungsaufwand abgetrennt und in der Regel verworfen werden muß. Dies gilt insbesondere für Methanol, das häufig Azeotrope bildet. Die Gewichtsausbeute, bezogen auf das Ketal, ist dadurch notwendigerweise vermindert.

Es bestand daher die Aufgabe, ein Kombinationsverfahren vorzuschlagen, das es erlaubt, einerseits den erforderlichen Enolether III über einem Heterogen-Katalysator in guten Ausbeuten herzustellen, ohne daß der aus dem Ketal stammende Alkohol als Koppelprodukt in stöchiometrischer Menge anfällt und das andererseits die Rückführung des bei den Reaktionen der Gleichungen 1 und 2 anfallenden Ketals in die Enoletherherstellung ermöglicht.

Diese Aufgabe wurde erfindungsgemäß gelöst mit einem Verfahren zur Herstellung von ungesättigten Ketonen der Formeln Ia bzw. Ib 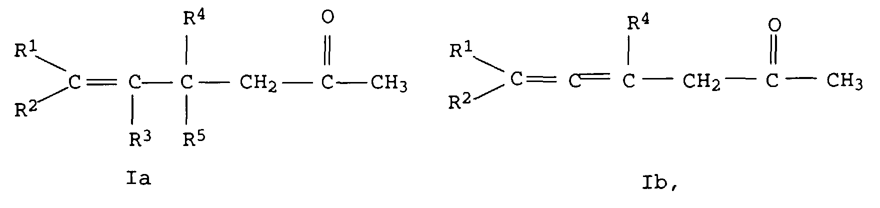 in denen R¹, R², R³, R⁴ und R⁵ Wasserstoff oder gegebenenfalls durch sauerstoffhaltige Gruppen substituierte Alkyl-, Alkenyl-, Cycloalkyl-, Cycloalkenyl-, Aryl- oder Aralkylreste bedeuten, wobei die Reste R¹ und R² auch zusammen einen 5- oder 6-gliedrigen Ring bilden können, gekennzeichnet durch die Kombination der folgenden Umsetzungen:
a) Die an sich bekannte Umsetzung von Allylalkoholen der Formel IIa bzw. Propargylalkoholen der Formel IIb 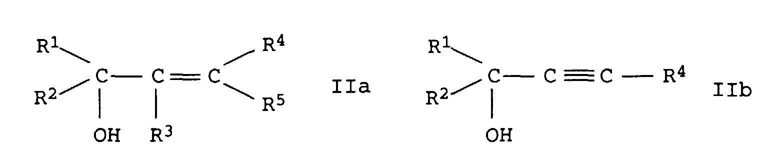 mit Isopropenylethern der Formel III 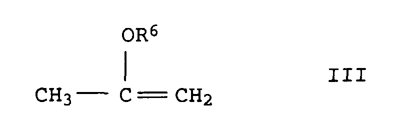 in der R⁶ für einen Alkylrest mit 1 bis 4 C-Atomen steht unter Bildung von Ketalen der Formel IV 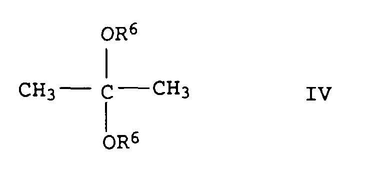 als Nebenprodukt, in der R⁶ die oben genannte Bedeutung hat,
b) Herstellung der Isopropenylether der Formel III durch Umsetzung von Ketalen der Formel IV mit Propin oder Allen oder deren Mischungen in der Gasphase bei erhöhter Temperatur in Gegenwart eines Zink oder Cadmium zusammen mit Silicium und Sauerstoff enthaltenden Heterogenkatalysators, und
c) Einspeisung des in der Reaktion (a) entstandenen Ketals der Formel IV in die Stufe (b) zur erneuten Herstellung des Isopropenylethers der Formel III.

Die Reaktion der Stufe (a) ist in der Fachliteratur eingehend beschrieben und wird im Rahmen der vorliegenden Erfindung nicht für sich, sondern nur in Kombination mit den Schritten (b) und (c) beansprucht.

Die Verfahrensbedingungen der Stufe (a) sind der DE 1193490 bzw. US 3,029 287 bzw. den genannten Veröffentlichungen in Helv. Chim.Acta c.c. zu entnehmen. Diesbezüglich wird auf die Angaben dieser Patentschriften und Literaturstellen ausdrücklich Bezug genommen und sie sollen als hier einkorporiert gelten.

Die Verfahrensbedingungen der Stufe (a) sind allgemein für die Ausführung des erfindungsgemäßen Kombinations-Verfahrens nicht kritisch und können selbstverständlich z.B. bei der Wahl des Katalysators und gegebenenfalls der Edukte auch variiert werden.

Demgemäß kommen selbstverständlich auch neuere Verbesserungen der Reaktion der Stufe a) in Betracht, wie sie z.B. in der DE 19 649 564.4 beschrieben sind.

Bevorzugte Ausgangsmaterialien der Formel IIa sind vor allem tertiäre Allylalkohole, wobei vorzugsweise R¹ einen gegebenenfalls durch sauerstoffhaltige Gruppen, z.B. Methoxy oder Ethoxy-Gruppen, substituierten gesättigten oder ungesättigten, verzweigten oder unverzweigten Alkylrest, einen Arylrest oder einen Alkylarylrest mit bis zu 20 C-Atomen und R² einen C1 bis C4 Alkylrest, insbesondere Methyl, bedeutet und wobei R¹ und R² auch zusammen einen gegebenenfalls durch eine oder mehrere niedere Alkylgruppen substituierten Tetramethylenrest oder Pentamethylenrest bedeuten können, und die Reste R³, R⁴, und R⁵ für Wasserstoff stehen. Im einzelnen seien 3-Methyl-1-buten-3-ol,3,7-Dimethyl-1-octen -3-ol (Hydrolinalool), 3,7-Dimethylocta-2,6-dien-3-ol (Linalool), 1-Vinyl-cyclohexanol, 3,7,11-Trimethyldodeca-1,6,10-trien-3-ol (Nerolidol), 3,7,11-Trimethyldodeca-1,6-dien-3-ol (Hydronerolidol) und 3,7,11-Trimethyldodec-1-en-3-ol(Tetrahydronerolidol) und Isophytol genannt.

Als Propargylalkohole kommen vorzugsweise tertiäre Propargylalkohole der Formel IIb in Betracht, in der R¹ für einen gegebenenfalls durch sauerstoffhaltige Gruppen substituierten gesättigten oder ungesättigten verzweigten oder unverzweigten Alkylrest, einen Arylrest oder einen Alkylarylrest, jeweils mit bis 20 C-Atomen und R² für einen C1 bis C4 Alkylrest, insbesondere Methyl, bedeuten und wobei R¹ und R² auch zusammen einen gegebenenfalls durch eine oder mehrere niedere Alkylgruppen substituierten Tetramethylenrest oder Pentamethylenrest bedeuten können und R⁴ für Wasserstoff steht.
Im einzelnen seien 3-Methylbutin-3-ol, 3,7-Dimethyl-1-octin-3-ol(Hydrodehydrolinalool), 3,7-Dimethylocta-6-en-1-in-3-ol(Dehydrolinalool), 3,7,11-Trimethyldodeca-6,10-dien-1-in-3-ol(Dehydronerolidol), 3,7,11-Trimethyldodeca-6-en-1-in-3-ol und 3,7,11-Trimethyldodec-1-in-3-ol genannt.

Als Isopropenylether der Formel III kommt insbesondere Isopropenylmethylether in Betracht.

Die Herstellung der Isopropenylether der Formel III 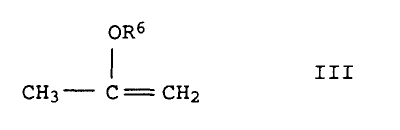 in der R⁶ die oben angegebene Bedeutung hat, wie sie in umfassenderer Form in der deutschen Patentanmeldung 19 726 667.3 beschrieben und beansprucht ist, erfolgt gemäß Stufe (b) durch Umsetzung von Ketalen der Formel IV 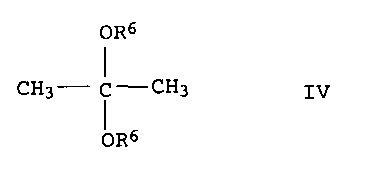 mit Propin und/oder Allen in der Gasphase in Gegenwart eines Zink oder Cadmium und Silicium und Sauerstoff enthaltenden Heterogenkatalysators.

Obgleich der Mechanismus dieser Reaktion nicht im einzelnen bekannt ist, kann die Reaktion formal so betrachtet werden, als ob ein Mol eines Alkohols R⁶OH aus der Dialkoxyverbindung der Formel IV auf das Acetylen bzw. Allen unter Bildung des Enolethers der Formel III übertragen wird.

Als Ketale kommen beispielsweise die Dimethyl-, Diethyl-, Di-n-propyl-, Di-n-butyl-, Di-i-butylketale von Aceton in Betracht. Besonders bevorzugt als Edukt ist 2,2-Dimethoxypropan (Acetondimethylketal).

Die Ketale und ihre Herstellung aus Aceton sind aus der Fachliteratur bekannt. Sie werden z.B. durch Umsetzung von Aceton mit den entsprechenden Alkoholen gewonnen oder bevorzugt durch Addition von Alkoholen an Alkine oder Allen, wobei man Gemische aus Enolethern und den entsprechenden Ketalen erhält, nach deren Auftrennung die Enolether unmittelbar verwendet und die Ketale der Stufe (b) zugeführt werden können.

In der Gesamtbilanz des erfindungsgemäßen Verfahrens sind jedoch nur die Verluste an Ketal durch Nebenreaktionen zu ersetzen, da in der Gesamtbilanz gemäß Gleichung 3,4 und Summengleichung 5 der erforderliche Enolether stets aus Propin und Allen nachgebildet wird.

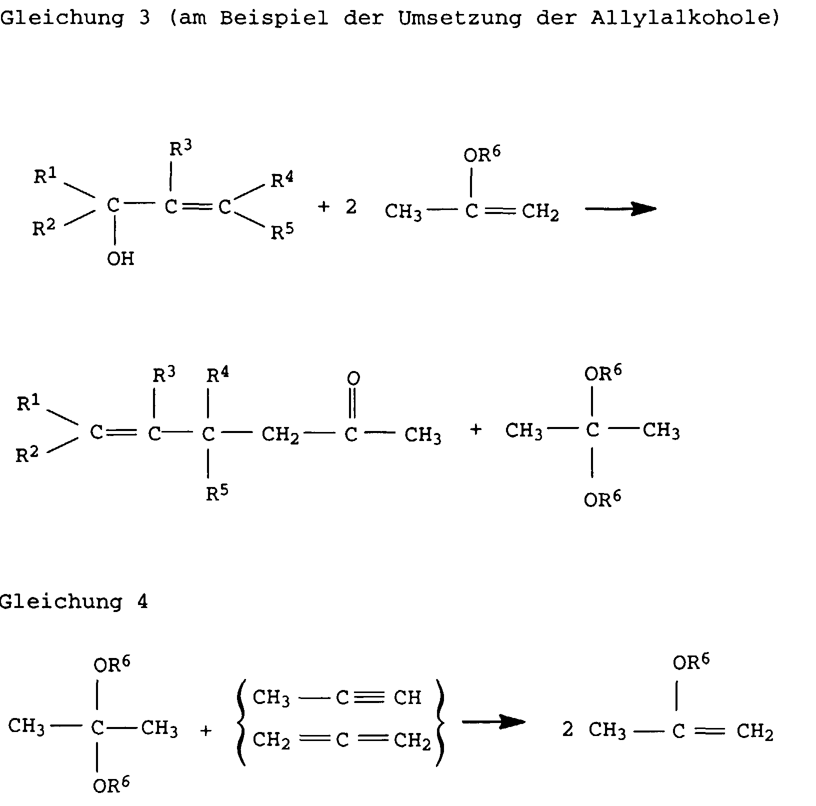

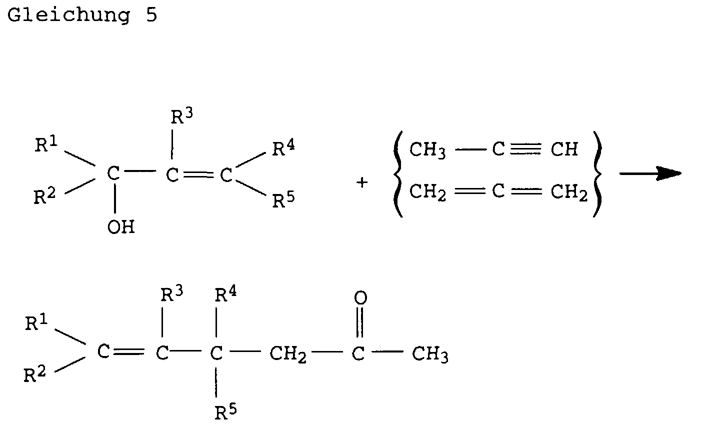

Anstelle von reinem Methylacetylen oder reinem Allen können auch deren Gemische verwendet werden, vor allem solche Gemische wie sie z.B. aus einem C₃-Strom eines Steamcrackers isoliert werden können.

Die Umsetzung der Ketale bzw. Acetale mit den Acetylenen bzw. Allenen erfolgt in Gegenwart des heterogen vorliegenden, Zink oder Cadmium und Silicium und Sauerstoff enthaltenden Katalysators in der Gasphase entweder über einem Festbett oder in einem Wirbelbett bei Temperaturen von 50 bis 400°C, vorzugsweise 100 bis 250°C und besonders bevorzugt 120 bis 200°C und Drücken von 0,1 bis 50 bar, insbesondere 0,8 bis 20 bar und besonders bevorzugt 0,9 bis 10 bar (alle Drücke bezogen auf die Summe der Partialdrücke der Edukte).

Gegebenenfalls kann das Reaktionsgemisch aus Gründen der Betriebssicherheit und der besseren Wärmeabfuhr mit Inertgasen wie Stickstoff, Argon, niedermolekularen Alkanen oder Olefinen verdünnt werden.

Das molare Verhältnis zwischen Ketal bzw. Acetal und Alkin bzw. Allen kann zwischen 0,01 und 100 liegen. Bevorzugt wird der Bereich zwischen 0,1 und 2, und besonders bevorzugt wird der Bereich zwischen 0,7 und 1,3 gewählt.

Als Zink oder Cadmium sowie Silicium und Sauerstoff enthaltende Katalysatoren kommen Cadmiumsilikate und bevorzugt Zinksilikate in Betracht, z.B. Silikate ausgewählt aus der Gruppe bestehend aus
(a) röntgenamorphen Zinksilikat oder Cadmiumsilikat, hergestellt durch Imprägnierung eines Kieselsäureträgers mit einem Zink- bzw. Cadmiumsalz,
(b) kristallinem Zinksilikat mit im wesentlichen der Zusammensetzung und Struktur des Hemimorphits der Formel Zn₄Si₂O₇(OH)₂·H₂O, wobei das Zink in einem bis zu 25%igen Unter- oder Überschuß, bezogen auf die stöchiometrische Zusammensetzung vorliegen kann und/oder
(c) im wesentlichen röntgenamorphen Zinksilikat, hergestellt durch Ausfällen in wäßriger Lösung aus einer löslichen Silicium- und Zinkverbindung, der Formel V

   ZnₐSi_{c}O_{a+2c-0,5e} (OH)ₑ · f H₂O V,

   in der e die Werte 0 bis zur Summe aus 2a+4c bedeutet, das Verhältnis a/c 1 bis 3,5 und f/a 0 bis 200 beträgt.

(a) Röntgenamorphe Zinksilikat- oder Cadmiumsilikat-Katalysatoren werden z.B. durch Beladen von amorpher Kieselsäure mit einem Zinksalz bzw. Cadmiumsalz und Formierung des Katalysators durch eine thermische Behandlung erhalten.
   Der SiO₂-Träger ist zumindest überwiegend amorph, hat eine BET-Oberfläche zwischen 10 und 1500 m²/g, besonders bevorzugt 100 bis 500 m²/g, eine Wasseraufnahmekapazität von 0,1 bis 2 ml/g, besonders bevorzugt von 0,7 bis 1,3 ml/g und kann als Pulver oder als fertiger Formkörper eingesetzt werden. Der Träger kann auch vor der Imprägnierung kalziniert werden. Bevorzugt wird der Träger aber nicht kalziniert.
   Als Zink- bzw. Cadmiumverbindung verwendet man eine in einem geeigneten Lösungsmittel lösliche Verbindung. Bevorzugt werden Zink(II)-Salze verwendet, die in Wasser oder wäßrigem Ammoniak oder Alkoholen, bevorzugt niederen Alkoholen, löslich sind und deren Zersetzungstemperatur unterhalb 400°C bis 500°C liegt.
   Besonders bevorzugt wird für die Imprägnierung eine ammoniakalkalische Zink(II)acetat-Lösung verwendet. In manchen Fällen hat es sich als vorteilhaft erwiesen, die Beladung mit Zink in mehreren aufeinander folgenden Tränkschritten vorzunehmen.
   Wenn der Träger als Pulver eingesetzt wird, kann der Katalysator durch Formgebung (z.B. durch Mischen, Kneten und Verstrangen oder Tablettieren) in die gewünschte Form gebracht werden.
   Zur Erhöhung des Porenvolumens können bei der Formgebung auch Porenbildner zugesetzt werden (z.B. Superabsorber wie Lutexal® P (Firma BASF Ludwigshafen) oder Walocel® (Methylcellulose/Kunstharz-Kombination, Firma Wolff, Walsrode)).
   Alternativ kann auch ein anderer Träger, z.B. Al₂O₃, mit einer Siliciumoxid-Vorläuferverbindung (z.B. Si(OR)₄) und mit einem Zinksalz oder Cadmiumsalz imprägniert werden.
   Die Zink- bzw. Cadmiumbeladung kann in weiten Grenzen variieren. Typische Werte für einen unkalzinierten Präkatalysator, der durch Tränkung eines SiO₂-Trägers mit einem Zinksalz bzw. Cadmiumsalz präpariert wurde, liegen z.B. zwischen 1 und 60 Gew.-% Zn oder Cd bevorzugt zwischen 7 und 30 Gew.-%. Besonders bevorzugt werden Gehalte zwischen 10 und 25 Gew.-% (jeweils berechnet als ZnO oder CdO). Der Präkatalysator kann außerdem mit anderen Elementen dotiert werden, bevorzugt mit Alkali-, Erdalkali- oder Übergangsmetallen. Ferner kann die katalytisch wirksame Komponente mit bis zu 80, vorzugsweise bis zu 50 und insbesondere bis zu 20 Molprozent noch mit weiteren Metallen dotiert sein, ausgewählt aus der Gruppe (A) bestehend aus Beryllium, Magnesium, Calcium, Strontium, Barium, Mangan, Eisen, Kobalt, Nickel und Kupfer, und der Gruppe (B), bestehend aus Titan, Zirkon, Hafnium, Germanium, Zinn und Blei, wobei die Elemente der Gruppe (A) teilweise Zink, bzw. Cadmium und die Elemente der Gruppe (B) teilweise Silicium ersetzen.
   Der Präkatalysator kann dann bei einer Temperatur von maximal 600°C, insbesondere zwischen 80 und 300°C, an Luft oder unter Inertgas kalziniert werden. Besonders bevorzugt ist die Kalzinierung zwischen 120 und 250°C an Luft.
   Nach der Herstellung des im allgemeinen katalytisch noch inaktiven Präkatalysators, durch Aufbringung einer Zink- oder Cadmium-Verbindung auf einen Siliciumoxid-Träger wird bevorzugt eine Formierung durchgeführt, bei der sich vor allem auf der Oberfläche des Katalysators die eigentliche Aktivphase ausbildet. Diese Festkörperreaktion wird durch die Anwesenheit von Wasser, Alkoholen, bevorzugt niederen Alkoholen oder Carbonsäuren, bevorzugt niederen Carbonsäuren gefördert und wird deshalb zweckmäßigerweise durch Erhitzen des Präkatalysators bei einer Temperatur zwischen 50 und 400°C in einer wasser- oder alkoholhaltigen Atmosphäre durchgeführt. Bevorzugt führt man die Reaktion zwischen 100 und 250°C in einem wasser- oder methanolhaltigen Gasgemisch aus. Besonders bevorzugt ist die Durchführung der Reaktion zwischen 120 und 200°C mit einem methanolhaltigen Gasgemisch direkt in dem Reaktor, in dem später die Umsetzung mit dem Alkin oder Allen stattfinden soll. Wenn von einem Präkatalysator auf der Basis von Zinkacetat ausgegangen wird, kann man sehr leicht bestimmen, wann die Festkörperreaktion beendet ist, da zu diesem Zeitpunkt fast kein Methylacetat im Abgas zu finden ist. In manchen Fällen hat es sich als vorteilhaft erwiesen, den Präkatalysator zur Ausbildung der Aktivphase unter Reaktionsbedingungen mit einem Gemisch aus Methanol mit Propin und Allen und eventuell auch noch anderen Komponenten (wie z.B. Propen oder Propan) zu beaufschlagen. Die Bildung der Aktivschicht wird durch das Ansteigen des Propin- und Allen-Umsatzes (nach ca. 5 bis 30 min, je nach Temperatur), durch das Ansteigen der Selektivität (nach 10 bis 300 min, je nach Temperatur) und durch das Abklingen der Konzentration von Methylacetat im Abgas angezeigt. Ein stationärer Zustand (mit hohen Propin- bzw. Allen-Umsätzen) und eine hohe Selektivität wird je nach Temperatur nach ca. 2 bis 20 Stunden erreicht.
   Gleichermassen können die entsprechenden Quecksilbersilikate hergestellt werden, die jedoch technisch und ökologisch weniger geeignet sind.
   Für die Charakterisierung der Katalysatorproben (frische, als auch Ausbauproben) wurden Standardmethoden verwendet. Die gemessene BET-Oberfläche, die typischerweise zwischen 10 und 800 m²/g liegt, sowie die Härte sind bei dem jeweiligen Beispiel angegeben. Bevorzugt werden Katalysatoren mit BET-Oberflächen zwischen 100 und 400 m²/g. Weiterhin wurden die Proben mittels Pulverröntgendiffraktometrie (XRD) und Transmissionselektronenmikroskopie (TEM) eingehend untersucht. Bei beiden strukturaufklärenden Methoden ist keine Fernordnung im Sinne einer kristallinen Struktur festzustellen, sämtliche Proben waren amorph. Die Verteilung des Zinks über den Träger wurde an entsprechenden Schnitten im Elektronenmikroskop sowie in der Mikrosonde untersucht. Sämtliche Proben zeigen, auch nach Ausbau, daß der Katalysator eine weitgehend homogene Elementverteilung besitzt und kein oder nur wenig kristallines ZnO enthält. In der IR-Untersuchung (KBr-Preßling) zeigt der mit Zinkacetat hergestellte aktive Katalysator keine Acetat-Banden (diese sind im Präkatalysator bei 1570, 1410, 670 und 610 cm⁻¹ noch sichtbar). Im ¹³C-CP-MAS-NMR sind auch keine Signale für Acetat mehr vorhanden. Im ²⁹Si-CP-MAS-NMR zeigt der Katalysator nur die breite Bande bei -109 ppm typisch für das amorphe SiO₂ und eine Schulter bei -99 ppm (ca. 10 % der Intensität des Hauptpeaks). Die Elementaranalyse eines Zn-Acetat/SiO₂-Präkatalysators zeigt, daß das molare Verhältnis C/Zn von der Kalzinierungstemperatur abhängt. Bei Raumtemperatur getrocknete Katalysatoren haben einen C/Zn-Verhältnis von 3,5 - 4. Nach der Kalzinierung bei 200 - 250°C (optimale Temperatur) liegt das C/Zn-Verhältnis zwischen 1 und 2. Bei höheren Temperaturen sinkt das C/Zn-Verhältnis noch weiter und ebenso die katalytische Aktivität der daraus gebildeten Katalysatoren. Nach Kalzinierung bei 500°C (24 Stunden) liegt das C/Zn-Verhältnis im Präkatalysator bei 0,02. Daraus kann kein aktiver Katalysator formiert werden. Da die Zersetzung des Zinkacetats auf dem Präkatalysator relativ langsam ist, kann dieser für kurze Zeiten sogar noch höheren Temperaturen ausgesetzt werden, ohne das die katalytische Aktivität vollständig verloren geht.
(b) Hemimorphit als Katalysator
   Hemimorphit ist ein Zinksilikat der Formel Zn₄Si₂O₇(OH)₂ · H₂O. Für die erfindungsgemäße Umsetzung sind jedoch nicht nur reiner Hemimorphit, sondern allgemein heterogene Katalysatoren geeignet, die zumindest überwiegend als Aktivkomponente Zinksilikat mit der Struktur des Hemimorphits der Formel Zn₄Si₂O₇(OH)_{2-2y}O_{y} · x H₂O enthalten, wobei x und y für die Werte 0 bis 1 stehen.
   Die Herstellung von Hemimorphit ist aus der Literatur bekannt. Sie kann unter Normalbedingungen oder hydrothermalen Bedingungen erfolgen.
   (b1) Herstellung unter Normalbedingungen
      A.G. Merkulov und B.S. Khristoforov, (Tr. Soveshch, Eksp. Tekh. Mineral. Petrogr., 8^{th} (1971), Meeting Date 1968, 322-8; Editor(s): V.V. Lapin; Publisher: "Nauka", Moscow, USSR) beschreiben die Herstellung verschiedener Zn-Silikate durch eine Reaktion von verschiedenen Zinksalzen (Carbonat, Sulfat, Chlorid, Acetat, Oxid) mit Na-Silikat und Natronlauge in wäßriger Lösung bei Temperaturen von 90 - 100°C und bei Normaldruck. Dabei bilden sich in Abhängigkeit vom eingestellten pH-Wert unterschiedliche Zinksilikate. So entsteht reiner Sauconit mit der Zusammensetzung Zn₃Si₄O₁₀(OH)₂ · n H₂O bei einem End-pH-Wert von 5 - 6. Reiner Willemit (α-Zn₂SiO₄) fällt im pH-Bereich von 6,5 - 8,5 an. Reiner Hemimorphit (Zn₄Si₂O₇(OH)₂ · H₂O) kristallisiert dagegen nur im schwach alkalischen Medium bei pH-Werten von größer 10 aus.
      In einer anderen Arbeit der genannten Autoren (A.G. Merkulov und B.S. Khristoforov, Izv. Sib. Otd. Akad. Nauk SSSR, Ser. Khim. Nauk (1969), (4), 70 - 4) wird angegeben, daß reiner Hemimorphit bei der Umsetzung von Zinksalzen mit Natriumsilikat und Natronlauge bei 90 - 100°C und Atmosphärendruck in wäßriger Lösung nur in einem pH-Bereich von 10 - 12 gebildet wird.
      Ferner konnten T. Baird, A.G. Cairns Smith und D.S. Snell (Reactivity of Solids, Proc. Int. Symp., 8th (1977), Göteborg, Meeting Date 1976, 337 - 42; Editor(s): J. Wood, O. Lindqvist und C. Helgesson; Publisher: Plenum Press, New York, N.Y.) große Kristalle von Hemimorphit durch Umsetzung von Zn(OH)₂ mit Kieselsäure und LiOH in wäßriger Lösung bei einem pH von 10 herstellen.
      Schließlich wurde von H. Nagata, M. Matsunage und K. Hosokawa (Zairyo-to-Kankyo (1993) 42, 225 - 233) Hemimorphit hergestellt, indem wäßrige Zinksulfat-Lösung mit Natronlauge und wäßriger Natriumsilikat-Lösung bei einem pH von 13 umgesetzt wurde, der erhaltene Niederschlag abgetrennt, ausgewaschen und bei 85°C mindestens 24 Stunden lang gealtert wurde.
   (b2) Hydrothermale Herstellung
      Gemäß EP 165 647 kann Hemimorphit aus einem säurebehandelten Tonmineral und Zinkoxid bzw. Zinkhydroxid unter hydrothermalen Bedingungen (170°C, 5 h) hergestellt werden. Die Säurevorbehandlung des Tones ist jedoch sehr aufwendig und deshalb ist dieses Verfahren nachteilig.
      Gemäß D.M. Roy und F.A. Mumpton (Econ. Geol. (1956) 51, 432 - 443) kann Hemimorphit ferner durch hydrothermale Umsetzung von Mischungen aus ZnO und SiO₂ bei 175 - 200°C gewonnen werden (Zusammensetzung: 3 ZnO + 2 SiO₂). Das erhaltene Produkt enthält überwiegend Hemimorphit, ist aber durch Sauconit (Zn₃Si₄O₁₀(OH)₂ · 4 H₂O) verunreinigt.
      Schließlich beschreiben P. Taylor und D.G. Owen, (Polyhedron (1984) 3(2) 151 - 155) die hydrothermale Synthese von Hemimorphit durch Umsetzung von ZnO mit SiO₂ in wäßriger Lösung bei 150°C. Zur Herstellung von Produkten mit einem hohen Hemimorphit-Anteil waren jedoch lange Reaktionszeiten von mindestens 4 Tagen erforderlich.
      Obgleich Hemimorphit-Produkte, die nach den oben beschriebenen bekannten Methoden erhalten wurden, sich als Katalysator für die erfindungsgemäße Addition sehr gut eignen, ergab sich, daß es wünschenswert war, deren Eigenschaften noch zu verbessern und eine Herstellungsmethode vorzuschlagen, mit deren Hilfe sich Katalysatoren mit reproduzierbar gutem Eigenschaftsprofil herstellen lassen.
      Demgemäß wird eine neue Herstellungsmethode sowohl unter Normaldruck als auch unter hydrothermalen Bedingungen bevorzugt, bei der man ein Alkali- oder Erdalkalisilikat, vorzugsweise Natriumsilikat mit einem Zinksalz, insbesondere Zinknitrat, und einer Base wie Alkali- oder Erdalkalihydroxid, insbesondere Natriumhydroxid in wäßriger Lösung bei pH-Werten von 4 bis 9,5 vorzugsweise 5,5 bis 8 und insbesondere in einem pH-Bereich um den Neutralpunkt, z.B. bei pH 6 bis 7,5, im Falle von Normaldruck bei Temperaturen von 50 bis 100°C, insbesondere 70 bis 100°C und im Falle von hydrothermalen Bedingungen bei Temperaturen von 100 bis 250°C, bevorzugt im Bereich von 100 bis 200°C umsetzt.
      Nach dieser Herstellungsmethode kann reiner Hemimorphit mit einem Zn/Si-Verhältnis von 2 synthetisiert werden. Es sind aber auch Hemimorphit-Präparate mit bis zu 25 % Unter- oder Überschuß an Zink, entsprechend einem Atomverhältnis Zn : Si von 1,6 bis 2,5 zugänglich. Als Katalysatoren werden Hemimorphite bevorzugt, die 0 bis 20 % Zinküberschuß enthalten. Besonders bevorzugt sind Hemimorphite, die 0 bis 10 % Zinküberschuß enthalten.
      Die Hemimorphit-Produkte fallen bei der Synthese als weißer kristalliner Niederschlag in Form einer wäßrigen Suspension an und müssen durch geeignete Maßnahmen, z.B. Filtration oder Zentrifugieren, von der wäßrigen Lösung getrennt werden. Im Falle der Filtration wird der erhaltene Filterkuchen daraufhin Natrium- und Nitrat-frei ausgewaschen und anschließend getrocknet. Die Trocknung kann bei Temperaturen bis 600°C erfolgen, wobei der bevorzugte Temperaturbereich 90 bis 250°C umfaßt. Thermogravimetrische Untersuchungen haben gezeigt, daß der auskristallisierte Hemimorphit der Zusammensetzung Zn₄Si₂O₇(OH)₂ · H₂O im Temperaturbereich von etwa 100 bis 200°C unter Beibehaltung der Hemimorphit-Struktur zunehmende Abteile seines Kristallwassers verliert, wobei Hemimorphit-Präparate der Zusammensetzung Zn₄Si₂O₇(OH)₂ · x H₂O mit x kleiner als 1 resultieren, wobei x mit steigender Temperatur abnimmt. Trocknet man in einem höheren Temperaturbereich von etwa 200 bis 600°C, so werden zusätzlich, ebenfalls unter Beibehaltung der Hemimorphit-Struktur, die im Hemimorphit enthaltenen OH⁻-Ionen in O²-Ionen und abgespaltenes H₂O umgewandelt (2 OH⁻ → H₂O + O²-), wobei Hemimorphit-Präparate der Zusammensetzung Zn₄Si₂O₇(OH)_{2-2y}O_{y} mit y = 0 bis 1 resultieren, wobei y mit steigender Temperatur zunimmt.
      Die nach der Trocknung bei Temperaturen bis 600°C, bevorzugt bei 90 bis 450°C erhaltene Hemimorphit-Präparate der Zusammensetzung Zn₄Si₂O₇(OH)_{2-2y}O_{y} · x H₂O, wobei x und y Werte von 0 bis 1 umfaßt, werden anschließend üblicherweise mit den gebräuchlichen Formgebungsverfahren, z.B. Tablettieren, Verstrangen oder als Schalenkatalysatoren auf Steatitkugeln zu katalytischen Formkörpern verarbeitet. Einzelheiten werden in den Beispielen beschrieben.
      Für die Charakterisierung der Katalysatorproben (frische Proben als auch Ausbau-Proben) wurden Standardmethoden verwendet. Die gemessene BET-Oberfläche liegt in der Regel zwischen 3 und 400 m²/g. Bevorzugt werden Katalysatoren mit BET-Oberflächen zwischen 20 und 300 m²/g verwendet. Weiterhin wurden die mit dem neuen Herstellungsverfahren erhaltenen Proben mittels Pulverröntgendiffraktometrie (XRD) und Transmissionselektronenmikroskopie (TEM) eingehend untersucht. Das gemessene Pulverröntgendiffraktogramm stimmt mit dem der Karteikarte 5-0555 der JCPDS-ICDD-Kartei (1995) überein.
(c) Röntgenamorpher Zinksilikat-Katalysator
   Verfährt man im wesentlichen unter gleichen Herstellungsbedingungen aber kürzerer Umsetzungszeit erhält man als Vorstufe zur Herstellung eines kristallinen Hemimorphits ein röntgenamorphes Produkt mit verbesserten katalytischen Eigenschaften.
   Dazu wird eine wäßrige Suspension eines Alkali- oder Erdalkalisilikats mit einer wäßrigen Lösung eines Zinksalzes bei
   a) Temperaturen von 20°C, bevorzugt 50°C bis zum Siedepunkt der sich ergebenden wäßrigen Suspension bei
   b) einem pH-Wert von 4 bis 9,5, bevorzugt bei einem pH-Wert in der Nähe des Neutralpunktes,
   c) und solchen Mengenverhältnissen von Alkalisilikat und Zinksalz umgesetzt, daß die Bedingungen der Formel V erfüllt werden und
   d) eine solche Verweilzeit eingehalten, daß noch nicht in erheblichem Maße Kristallisation des Zinksilikats eintritt.

   Das so erhältliche im wesentlichen röntgenamorphe Zinksilikat enthält Zn²⁺-, Si⁴⁺ und O²-Ionen; darüber hinaus kann die Verbindung OH-Ionen und Hydratwasser enthalten. Das Zn/Si-Verhältnis beträgt 0,3 bis 5, bevorzugt 1 bis 2,7, besonders bevorzugt 2 bis 2,3 und ganz besonders bevorzugt 2. In letzterem Fall weist das röntgenamorphe Zinksilikat damit das Zn/Si-Verhältnis des kristallinen Hemimorphits (Zn₄Si₂O₇(OH)₂·H₂O) auf. Das unter Anwendung von Cu-Kα₁-Strahlung (λ = 1,5406 Å) erhaltene Pulver-Röntgenbeugungsdiagramm des röntgenamorphen Zinksilikats weist in einem Diagramm, bei dem die Intensität A der gebeugten Röntgenstrahlung als Funktion des zweifachen Beugungswinkels (2θ) aufgetragen ist, im 2θ-Bereich von 10° bis 90° sehr breite Intensitätsmaxima bei 2θ = 31⁰ ± 5° und bei 2θ = 61° ± 7° auf.

Auch der erfindungsgemäß zu verwendende amorphe Zinksilikat-Fällungskatalysator kann mit bis zu 80, vorzugsweise bis zu 50 und insbesondere bis zu 20 Molprozent noch mit weiteren Metallen dotiert sein ausgewählt aus der Gruppe (A) bestehend aus Beryllium, Magnesium, Calcium, Strontium, Barium, Mangan, Eisen, Kobalt, Nickel, Kupfer, Cadmium und Quecksilber und der Gruppe (B), bestehend aus Titan, Zirkonium, Hafnium, Germanium, Zinn und Blei, wobei die Elemente der Gruppe (A) teilweise Zink und die Elemente der Gruppe (B) teilweise Silicium in der Hemimorphit-Struktur ersetzen.

Das röntgenamorphe Zinksilikat fällt bei der Herstellung als Pulver an. Dieses Pulver kann als solches für die katalytische Umsetzung eingesetzt werden (z.B. in einem Wirbelbettreaktor) oder nach Verformung (z.B. Verstrangung, Tablettierung, etc., evtl. auch unter Zusatz von Hilfsstoffen) in einer für einen Festbettreaktor geeigneten Form.

Vor dem Einsatz kann der Katalysator bei Temperaturen zwischen 80°C und 750°C kalziniert werden. Bevorzugt wird der Katalysator zwischen 120°C und 500°C kalziniert. Besonders bevorzugt ist die Kalzinierung zwischen 200° und 400°C an der Luft. Zur Erhöhung des Porenvolumens können bei der Formgebung z.B. beim Tablettieren oder Verstrangen auch Porenbildner zugesetzt werden (z.B. Super-absorber wie Lutexal P® (Firma BASF AG) oder Walocel® (Methylcellulose/Kunstharz-Kombination, Firma Wolff, Walsrode AG)).

Allgemeine Reaktionsbedingungen des erfindungsgemäßen Kombinationsverfahrens mit den Schritten (a), (b) und (c).

Die Kombination der Stufen (a) bis (c) kann diskontinuierlich und vorzugsweise kontinuierlich durchgeführt werden. Im Falle der diskontinuierlichen Durchführung wird das in der Stufe (a) als Nebenprodukt anfallende Ketal der Formel IV in einem Vorratsbehälter gesammelt und zeitlich und gegebenenfalls räumlich vom Verfahren der Stufe (a) getrennt zum Enolether der Formel III umgesetzt.
Bei der im technischen Maßstab bevorzugten kontinuierlichen Durchführung ist die Enoletherherstellung und die Rückführung des Ketals in die Enolether-Herstellungsstufe in einer Anlage räumlich integriert. Für die Pufferbehälter wird dann nur ein sehr geringes Volumen benötigt und ein Transport entfällt.

Die Reaktion der Stufe (b) wird in der Regel in einem Rohrreaktor durchgeführt, wobei man das Propin und/oder Allen (oder vorzugsweise ein Gemisch der Zusammensetzung 30 - 43 Vol.-% Propin, 16 - 20 Vol.-% Allen, 20 - 45 Vol.-% Propen, 5 - 10 Vol.-% Isobutan und 2 - 6 Vol.-% Propan- erhalten durch Destillation aus einem Seitenstrom eines Steamcrackers) mit dem Ketal unter Druck zudosiert. Dabei werden die Edukte kurz vor dem Reaktor gemischt und in den Reaktorraum entspannt.

Die Reaktion wird in der Regel isotherm bei Temperaturen von 120 bis 300°C und einer Zulaufrate von 0,5 bis 10 mmol/min Propin und/oder Allen und 0,5 bis 20 mmol/min Ketal durchgeführt. Der Reaktionsdruck beträgt 0,1 bis 50 vorzugsweise 1,1 bis 3,5 bar (absolut).

Der in der Stufe (b) gebildete Enolether wird von dem Propen/Propan-Inertgasstrom abgetrennt und dann durch Destillation gereinigt. Der destillierte Enolether wird dann in Stufe (a) eingesetzt.

Das in der Stufe (a) als Nebenprodukt entstandene Ketal wird aus dem Reaktionsaustrag, gegebenenfalls nach vorheriger Neutralisation durch Destillation über eine Kolonne mit ca. 10 theoretischen Böden bei einem Druck von 100 mbar bis 1 bar, bevorzugt 300 - 800 mbar über Kopf abgetrennt. Im Destillat befinden sich neben dem Ketal IV überschüssiger Enolether III, sowie Aceton. Dieser Strom kann gemäß Stufe (c) direkt in die Synthese des Enolethers eingespeist werden, oder aber bevorzugt in einer weiteren Kolonne nochmals aufgereinigt werden. Diese Kolonne kann diskontinuierlich oder bevorzugt kontinuierlich betrieben werden. In diesem Fall wird der überschüssige Enolether III als Kopfprodukt gewonnen und direkt als Edukt für Stufe (a) verwendet. Aceton wird über einen Seitenabzug ausgeschleust. Das Ketal IV fällt im Sumpf an und wird gemäß Stufe (c) mit Propin/Allen zum Enolether III umgesetzt.

### Beispiele

### Beispiel 1

### Stufe a)

In ein Druckgefäß werden 200 g Hydrodehydrolinalool (1,3 mol), 306 g eines Azeotropes bestehend aus 2-Methoxypropen (hergestellt gemäß dem Verfahren der Stufe b s.u.) und Methanol (3.9 mol 2-Methoxypropen) und 1 g Kaliumhydrogensulfat (getrocknet und pulverisiert) eingefüllt. Der Reaktor wurde mit Stickstoff gespült und verschlossen. Während des Aufheizens sprang die Reakton an. Die Temperatur stieg für ca. 20 Minuten auf 140°C, der Druck auf ca. 5 bar an. Nun rührte man die Reaktionsmischung noch 2.5 h bei 120°C zur Vervollständigung des Umsatzes. Zuerst wurden die im Reaktoraustrag enthaltenden leichtsiedenden Komponenten, d. s. in erster Linie 2,2-Dimethoxypropan und überschüßiges 2-Methoxypropen, an einem Rotationsverdampfer bei ca. 500 mbar abgetrennt und in einer Kühlfalle kondensiert. Der Rückstand wurde im Vakuum bei 1 mbar über eine Brücke destilliert. Man erhielt 249 g 6,10-Dimethylundeca-4,5-dien-2-on in einer Reinheit von 85 %.

### Stufe c)

Das 2,2-Dimethoxypropan, Aceton und überschüssiges 2-Methoxypropen enthaltende Kondensat aus mehreren Ansätzen wurde fraktioniert destilliert und die aus 2-Methoxypropen bestehende Fraktion für die Stufe (a) unmittelbar wiederverwendet. Die aus 2,2-Dimethoxypropan bestehende Fraktion wurde in die Stufe (b) eingespeist. Die hauptsächlich aus Aceton bestehende Fraktion wurde verworfen.

### Stufe b)

### ba) Katalysatorherstellung (amorphes Zinksilikat; durch Imprägnierung)

Der Zn/SiO₂-Trägerkatalysator wurde durch Imprägnierung von röntgenamorphen SiO₂-Formkörpern (Kugeln mit ⌀ 3 - 6 mm) mit einer BET-Oberfläche von 358 m²/g, Wasseraufnahmekapazität von 0,9 ml/g und Härte von 43 N/Formkörper mit ammoniakalischer Zinkacetatlösung erhalten. Dazu wurden 225 g SiO₂-Träger (Siligel, Fa. Solvay) mit 151,70 g Zn(OAc)₂ · 2 H₂O (Merck), gelöst in 220 g 9 %iger NH₄OH-Lösung bei Raumtemperatur, getränkt, der Präkatalysator anschließend für 16 h bei 120°C getrocknet und dann bei 250°C für 4 h unter Luft kalziniert. Der Präkatalysator wies eine BET-Oberfläche von 195 m²/g und eine Härte von 76 N/Formkörper auf. Das Acetat/Zn-Verhältnis lag bei 0,9 mol/mol.

### bb) Umsetzung

In einem Rührreaktor wurden ca. 90 ml des Präkatalysators eingefüllt. Propin/Allen-Gemisch (55 mol-%ig, Rest Propen) und 2,2-Dimethoxypropan wurden dann mittels HPLC-Pumpen zudosiert. Die Reaktion wurde bei der ersten Einstellung (170°C) solange fortgeführt, bis der aktive Katalysator vollständig gebildet war und Umsatz und Selektivität konstant waren (ca. 20 Stunden). Danach wurde die Temperatur und die Zuläufe gemäß Tabelle 1 geändert. Die Ergebnisse sind in Tabelle 1 zusammengefaßt. Der Druck betrug bei allen Versuchen 1,35 bar (abs). Abkürzungen: 2MP: 2-Methoxypropen; 22DMP: 2,2-Dimethoxypropan; 1MP: 1-Methoxypropen (cis und trans); 11DMP: 1,1-Dimethoxypropan. Die angegebenen Selektivitäten beziehen sich auf Propin und Allen.

Die kondensierten Reaktionsausträge wurden fraktioniert destilliert. Die 2-Methoxypropen-Fraktion wurde in der Stufe (a) als Edukt verwendet und die nicht umgesetzte Dimethoxypropan-Fraktion in die Umsetzung (bb) zurückgeführt.

**Tabelle 1**

| Vers. -Nr. | Temp. C° | Zuläufe/mol/min | | | Umsätze/% | | Selektivitäten/% | | | |
|---|---|---|---|---|---|---|---|---|---|---|
| | | Propin/Allen | 22DMP | Gesamt | Propin/Allen | 22DMP | 2MP | Aceton | 1MP | 11DMP |
| 1.1 | 170 | 2,31 | 2,17 | 6,20 | 69 | 90 | 95 | 1 | 2 | <1 |
| 1.2 | 150 | 1,36 | 1,57 | 4,26 | 57 | 69 | 96 | 1 | 2 | <1 |
| 1.3 | 130 | 1,13 | 1,11 | 3,31 | 44 | 59 | 96 | 1 | 2 | <1 |

### Beispiel 2

### Stufe a)

In ein Druckgefäß wurden 190 g 3,7,11-Trimethyldodec-1-in-3-ol (Reinheit 95 %, 0,848 mol) und 200 g 2-Methoxypropen (2,78 mol)sowie 0,58 g KHSO₄ gelöst in 1.74 g Wasser zugegeben. Der Reaktor wurde mit Stickstoff gespült und verschlossen. Nach einer Reaktionszeit von 1 h bei 120°C wurde auf ca. 50°C abgekühlt und nochmals 0.58 g KHSO₄ gelöst in 1.74 g Wasser zugegeben. Nun wurde nochmals auf 120°C aufgeheizt. Nach einer Stunde Reaktionszeit ist der Umsatz vervollständigt. Wie in Beispiel 1 wird destilliert. Man erhält 147 g eines Gemisches bestehend hauptsächlich aus 2-Methoxypropen und 2,2-Dimethoxypropan und 213 g einer Fraktion bestehend aus 1.6 % 3,7,11-Trimethyldodec-1-in-3-ol, 67 % 6,10,14-Trimethylpentadeca-4,5-dien-2-on, und 7.3 % 6,10,14- Trimethylpentadeca-3,5-dien-2-on.

### Stufe c)

Das Methoxypropen/2,2-Dimethoxypropan Aceton-Gemisch aus mehreren Ansätzen wird fraktioniert destilliert , die Methoxypropen-Fraktion unmittelbar für die Reaktion der Stufe (a) wiederverwendet und die Dimethoxypropan-Fraktion in die Stufe b (s.u.) als Edukt eingeführt.

### Stufe b)

### ba) Katalysatorherstellung (Hemimorphit Zn/Si = 2)

In einem 8-l-Rührbehälter stellte man aus 4,5 l vollentsalztem Wasser und 145,1 g pulverförmigem Natronwasserglas mit einem SiO₂-Gehalt von 62,1 Gew.-% und einem Na₂O-Gehalt von 19,0 Gew.-% (Fa. Riedel-de Haen, D-30918 Seelze) eine Suspension A mit 1,5 Mol SiO₂ und 0,89 Mol Na her. Ferner wurden 910,7 g Zn(NO₃)₂ · 6 H₂O (98 %ig) in 2,25 l vollentsalztem Wasser bei Raumtemperatur gelöst, wobei eine Lösung B mit 3 Mol Zn und 6 Mol NO₃ erhalten wurde. Schließlich wurde eine wäßrige Lösung aus 204,4 g NaOH in 0,225 l vollentsalztem Wasser hergestellt, wobei eine Lösung C mit einem NaGehalt von 5,11 Mol erhalten wurde. Nun wurden die Lösungen B und C bei Raumtemperatur in die Suspension A gegeben, wobei sich eine milchige Suspension D mit folgenden Elementanteilen ergab: Zn-Gehalt = 3 Mol, Si-Gehalt = 1,5 Mol, Na-Gehalt = 6 Mol, NO₃-Gehalt = 6 Mol. Der pH-Wert der erhaltenen Suspension D betrug 7,1. Die Suspension D wurde auf 90°C aufgeheizt und bei dieser Temperatur 24 Stunden lang mit einer Umdrehungszahl von 200 Upm gerührt. Die Suspension wurde anschließend auf Raumtemperatur abgekühlt und ein End-pH-Wert von 7,0 gemessen. Der auskristallisierte weiße Niederschlag wurde abfiltriert und mit vollentsalztem Wasser Na-frei gewaschen und der anfallende Filterkuchen bei 90°C in einem Trockenschrank getrocknet.

Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit der Herstellung von Zn₄Si₂O₇(OH)₂ · H₂O anzeigte. Die nach BET bestimmte spezifische Oberfläche des erhaltenen Pulvers betrug 30 m²/g.

Zur Herstellung eines Katalysators wurde das noch feuchte Pulver direkt zu Strängen verpreßt (⌀ = 3 mm, Preßdruck = 50 bar), die anschließend 16 h bei 120°C getrocknet wurden. Der fertige Katalysator hatte eine BET-Oberfläche von 26 m²/g und eine Härte von 6 N/Formkörper.

### bb) Umsetzung

In einen Rührreaktor wurden ca. 90 ml des Katalysators eingefüllt. Propin/Allen-Gemisch (ca. 63 vol-%ig, 1,68 mmol/min) und 2,2-Dimethoxypropan (2,17 mmol/min; Gesamtzulauf mit Inerten: 6,46 mmol/min; Verhältnis 2,2-Dimethoxypropan/(Propin+Allen) = 1,29) wurden dann mittels HPLC-Pumpen zudosiert. Die Reaktionstemperatur betrug 170°C und der Druck 1,35 bar (abs), der Partialdruck der Edukte betrug 0,8 bar. Es wurden von Anfang an (d.h. der Katalysator hat keine Formierungszeit) folgende Selektivitäten beobachtet: 2-Methoxypropen: 97,4 %; Aceton: 2,3 %; cis und trans 1-Methoxypropen: 0,3 %.

Die kondensierten Reaktionsausträge wurden fraktioniert destilliert. Die 2-Methoxypropen-Fraktion wurde in der Stufe (a) als Edukt eingesetzt und die nicht zugesetzte Dimethoxypropan-Fraktion in die Umsetzung (bb) zurückgeführt. In geringen Mengen entstandenen Nebenprodukte wurden verworfen.

### Beispiel 3

### Stufe a)

25.9 g Hydrolinalool und 45.5 g Isopropenylethylether (Reinheit 85 %) und 93 mg 75 %ige Phosphorsäure wurden in einen Druckbehälter mit einem Volumen von 300 ml eingefüllt, der Behälter wurde verschlossen, mit Stickstoff gespült und auf 175°C aufgeheizt. Nach einer Reaktionszeit von 12 h wurde abgekühlt, der Behälter entleert und der Austrag über eine Kolonne (h=10 cm, gefüllt mit 3 mm Maschendrahtringen) destilliert. Die erste Fraktion (27.2 g) hatte bei Normaldruck eine Übergangstemperatur von 70°C und enthielt als Hauptprodukt 2,2-Diethoxypropan. Bei 0.9 mbar erhielt man 6,10-Dimethylundec-5-en-2-on in einer Ausbeute von 83 %.

### Stufe c)

Der 2,2-Diethoxypropan enthaltende Vorlauf wurde aus mehreren Ansätzen gesammelt und als Edukt der Stufe b) eingeführt.

### Stufe b)

### ba) Katalysatorherstellung (Hemimorphit Zn/Si = 2,2)

In einem 6 l Rührbehälter wurde aus 3,0 l vollentsalztem Wasser und 96,8 g pulverförmigem Natronwasserglas mit einem SiO₂-Gehalt von 62,1 Gew.-% und einem Na₂O-Gehalt von 19,0 Gew.-% (Fa. Riedel-de Haen, D-30918 Seelze) eine Suspension A mit 1,0 Mol SiO₂ und 0,59 Mol Na und aus 667,8 g Zn(NO₃)₂ · 6 H₂O (98 %ig) in 1,5 l vollentsalztem Wasser bei Raumtemperatur eine Lösung B mit 2,2 Mol Zn und 4,4 Mol NO₃ sowie eine wäßrige Lösung C mit einem Na-Gehalt von 3,81 Mol aus 152,3 g NaOH in 0,4 l vollentsalztem Wasser hergestellt. Die Lösungen B und C wurden in die Suspension A bei Raumtemperatur gegeben, wobei sich eine milchige Suspension D mit folgenden Elementanteilen ergab: Zn-Gehalt = 2,2 Mol, Si-Gehalt = 1 Mol, Na-Gehalt = 4,4 Mol, NO₃-Gehalt = 4,4 Mol. Der pH-Wert der erhaltenen Suspension D betrug 7,2. Die Suspension D wurde auf 90°C erhitzt und bei dieser Temperatur 24 Stunden lang mit einer Umdrehungszahl von 200 Upm gerührt. Nach Abkühlen der Suspension auf Raumtemperatur wurde ein End-pH-Wert von 7,0 gemessen. Der angefallene weiße Niederschlag wurde abfiltriert, mit vollentsalztem Wasser Na-frei gewaschen und der erhaltene Filterkuchen bei 90°C in einem Trockenschrank getrocknet.

Das getrocknete weiße Pulver wurde röntgenographisch untersucht, wobei sich ein Röntgenpulverdiagramm ergab, das dem der Karteikarte 5-0555 der JCPDS-ICDD-Kartei (1995) voll entsprach und damit die Herstellung von Zn₄Si₂O₇(OH)₂ · H₂O anzeigte. Die nach BET bestimmte spezifische Oberfläche des erhaltenen Pulvers betrug 60 m²/g.

650 g des Pulvers wurden mit 20,2 g Magnesiumstearat (Merck) vermischt und zu 20 mm Tabletten gepreßt. Diese Tabletten wurden zu Splitt (< 0,5 mm) verarbeitet. Die Tabletten wurden dann 10 Stunden bei 350°C kalziniert. Der fertige Katalysator hatte eine BET-Oberfläche von 44 m²/g und eine Härte von 44 N/Formkörper.

### bb) Umsetzung

In einen Rührreaktor wurden ca. 90 ml des Katalysators eingefüllt. Propin/Allen-Gemisch (ca. 60 vol-%ig, 1,64 mmol/min) und 2,2-Diethoxypropan (2,14 mmol/min; Gesamtzulauf mit Inerten: 8,34 mmol/min; Verhältnis 2,2-Diethoxypropan/(Propin+Allen) = 1,30) wurden dann mittels HPLC-Pumpen zudosiert. Die Reaktionstemperatur betrug 170°C und der Druck 1,35 bar (abs.), der Partialdruck der Edukte betrug 0,8 bar. Es wurden von Anfang an (d.h. der Katalysator hat keine Formierungszeit) folgenden Selektivitäten beobachtet: 2-Ethoxypropen: 97,0 %; Aceton: 2,3 %; cis und trans 1-Ethoxypropen: 0,5 %.

Für den Katalysator wurden nach dem Ausbau die folgenden Werte ermittelt: BET 44 m²/g, Härte von 12 N/Formkörper.

Der gesammelte kondensierte Reaktionsaustrag wurde fraktioniert destilliert und die 2-Ethoxypropen-Fraktion als Edukt der Stufe a) zugeführt. Nicht umgesetztes Diethoxypropan wurde in die Reaktion zurückgeführt.

### Beispiel 4

### Stufe a)

18.7 g Hydrolinalool, 46 g Isopropenylpropylether (Reinheit 80 %) und 85 mg 75 %ige Phosphorsäure wurden wie im vorigen Beispiel zur Reaktion gebracht und aufgearbeitet. Man erhielt 27 g einer Nebenprodukt-Fraktion mit dem Hauptbestandteil 2,2 Dipropoxypropan und eine Ausbeute von 79 % d.Th. 6,10-Dimethylundec-5-en-2-on, bezogen auf Hydrolinalool.

Die 2,2-Dipropoxypropan-Fraktion wurde in die Stufe (b) eingespeist.

### Stufe b)

### (Röntgenamorpher Fällungskatalysator; Zn/Si-Verhältnis 2,1)

In einem 12-l-Rührbehälter wurden unter ständigem Rühren (100 Upm) in 7,5 l vollentsalztem und 80°C warmem Wasser 120,93 g pulverförmiges Natronwasserglas mit einem SiO₂-Gehalt von 62,1 Gew.-% und einem Na₂O-Gehalt von 19,0 Gew.-% (Fa. Riedel-deHaen, D-30918 Seelze) gegeben, wobei eine Suspension A mit 1,25 Mol SiO₂ und 0,74 Mol Na erhalten wurde. Anschließend wurde eine wäßrige Lösung B aus 180,4 g NaOH (entspricht 4,51 Mol Na) in 0,5 l vollentsalztem Wasser hergestellt. Außerdem wurden 796,8 g Zn(NO₃)₂ · 6 H₂O (Zn-Gehalt = 98 %) in 2,5 l vollentsalztem Wasser gelöst, wobei eine Lösung C mit 2,625 Mol Zn und 5,25 Mol NO₃ erhalten wurde. Daraufhin wurde die Lösung B in die 80°C warme Suspension A gegeben, wobei nach ca. 5 Minuten eine klare Lösung D erhalten wurde. In die erhaltene Lösung D wurde anschließend die Lösung C gegeben. Dabei ergab sich eine weiße Suspension E mit einem Zn-Gehalt von 2,625 Mol, einem Si-Gehalt von 1,25 Mol, einem Na-Gehalt von 5,25 Mol und einem NO₃-Gehalt von 5,25 Mol. Die Suspension E wurde bei 80°C 2 Stunden lang unter Rühren (100 Upm) erhitzt und anschließend auf Raumtemperatur abgekühlt. Es wurde nach Abkühlung ein End-pH-Wert von 6,5 gemessen. Der angefallene weiße Niederschlag wurde abfiltriert und mit vollentsalztem Wasser Na-frei gewaschen. Der erhaltene Filterkuchen wurde bei 80°C in einem Trockenschrank getrocknet.

Das getrocknete weiße Pulver wurde röntgenographisch untersucht, wobei sich ein Röntgenpulverdiagramm ergab, das dem der Fig. 2 entspricht und damit die Herstellung von überwiegenden Mengen an röntgenamorphem Zinksilikat neben einer geringen Menge an kristallinem ZnO (Karteikarte 5.0664 der JCPDS-ICDD-Kartei (1995)) anzeigte. Die nach BET bestimmte spezifische Oberfläche des erhaltenen Pulvers betrug 102,1 m²/g.

650 g des wie oben beschrieben hergestellten amorphen Zinksilikats mit der Zusammensetzung von Hemimorphit wurde wurden mit 20,2 g Zn-Stearat vermischt und zu 20-mm-Tabletten vorkompaktiert, anschließend zu Splitt mit einem Durchmesser von < 0,5 mm zerkleinert und dann zu Tabletten mit dem Format 4,75 x 5,2 mm verformt. Der Katalysator besaß eine BET-Oberfläche von 75 m²/g und eine Härte von 43 N/Tablette. Eine 100 g Portion des Katalysators wurde dann bei 350°C für 10 Stunden an der Luft kalziniert.

In einen Rührreaktor wurden ca. 90 ml des Katalysators eingefüllt. Propin/Allen-Gemisch (49,8 vol.-%, 1,84 mmol/min) und 2,2-Dipropoxypropan (2,15 mmol/min; Gesamtzulauf mit Inerten: 6,79 mmol/min; Verhältnis 2,2-Dipropoxypropan/(Propin+Allen) = 1,17) wurden dann mittels HPLC-Pumpen zudosiert. Die Reaktionstemperatur betrug 170°C und der Druck 1,35 bar (abs), der Partialdruck der Edukte 0,8 bar. Es wurden folgende Selektivitäten beobachtet: 2-Propoxypropen 97,1 %; 2-Dipropoxypropan 2,5 %, cis- und trans-1-Propoxypropen 0,4 %. Der Umsatz, bezogen auf Propin/Propadien, betrug 21 %.

Dieser Katalysator zeigt praktisch keine Formierungszeit. Der angegebene Umsatz und die Selektivität waren von Anfang an quasi gleichbleibend. Die BET-Oberfläche betrug nach der Kalzinierung 82 m²/g und nach dem Ausbau 64 m²/g. Die Härte betrug nach der Kalzinierung 28 N/Tablette und nach dem Ausbau 36 N/Tablette.

### Beispiel 5

380.8 g Hydrolinalool, 55,5 g Isopropenyl-n-Butylether (Reinheit 90 %) und 113 mg 75 %ige Phosphorsäure wurden wie im Beispiel 4 zur Reaktion gebracht. Die Aufarbeitung erfolgte durch Destillation bei 0.3 mbar. Man erhielt 29 g eines Gemisches bestehend hauptsächlich aus 2,2-Dibutoxypropan, Isopropenyl-n-Butylether und Butanol, 6,10 Dimethylundec-5-en-2-on wurde in einer Ausbeute von 74 % erhalten.

Unter Verwendung der in Beispiel 4 beschriebenen Katalysators erhielt man bei Rückführung des 2,2-Dibutoxypropans und Umsetzen mit frischem 2,2-Dibutoxypropans ähnliche Ergebnisse.

### Beispiel 6

66 g Hydronerolidol, 60 g eines Azeotropes aus Isopropenylmethylether und Methanol (ca. 91 % Isopropenylmethylether) und 248 mg 75 %ige Phosphorsäure wurden in einem Druckgefäß vereinigt. Nach Spülen mit Stickstoff wurde die Reaktionsmischung 8 h bei 150°C gerührt. Der Reaktoraustrag wurde mit Aluminiumtriisopropylat neutralisiert und fraktioniert destilliert. Bei Normaldruck erhielt man 42 g eines Gemisches, bestehend aus 85 % 2,2-Dimethoxypropan, 11.3 % Aceton, 0.4 % Isopropenylmethylether und 0.4 % Methanol. Bei 0.3 mbar erhielt man 6,10,14-Trimethylpentadeca-5,9-dien-2-on in einer Ausbeute von 78 % bezogen auf eingesetztes Hydronerolidol.

Verfährt für die Herstellung des 2-Methoxypropens wie in Beispiel 1 beschrieben und führt das als Nebenprodukt gebildete 2,2-Dimethoxypropan nach dessen Abtrennung von Aceton in die Stufe (b) zurück, erhält man ähnliche Ergebnisse.

## Patentansprüche

1. Verfahren zur Herstellung von ungesättigten Ketonen der Formeln Ia bzw. Ib 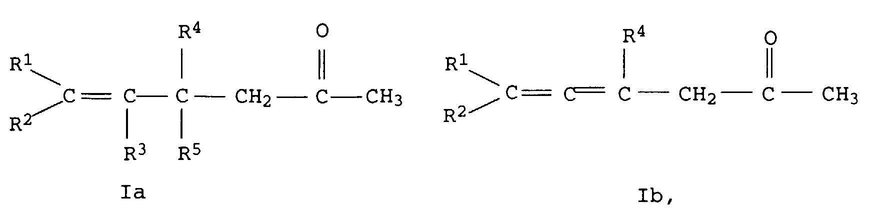 In denen R¹, R², R³, R⁴ und R⁵ Wasserstoff oder gegebenenfalls durch sauerstoffhaltige Gruppen substituierte Alkyl-, Alkenyl-, Cycloalkyl-, Cycloalkenyl-, Aryl- oder Aralkylreste bedeuten, wobei die Reste R¹ und R² auch zusammen einen 5- oder 6-gliedrigen Ring bilden können, gekennzeichnet durch die Kombination der folgenden Umsetzungen:
a) Die an sich bekannte Umsetzung von Allylalkoholen der Formel IIa bzw. Propargylalkoholen der Formel IIb 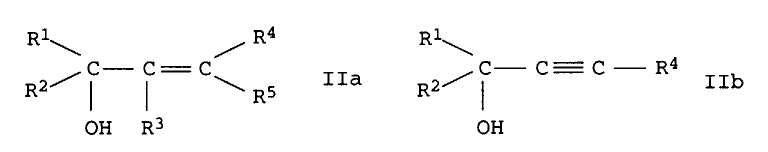 mit Isopropenylethern der Formel III 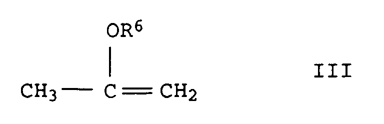 in der R⁶ für einen Alkylrest mit 1 bis 4 C-Atomen steht unter Bildung von Ketalen der Formel IV 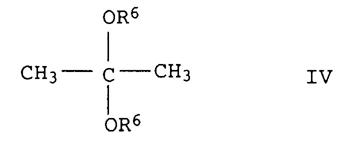 als Nebenprodukt, in der R⁶ die oben genannte Bedeutung hat,
b) Herstellung der Isopropenylether der Formel III durch Umsetzung von Ketalen der Formel IV mit Propin oder Allen oder deren Mischungen in der Gasphase bei erhöhter Temperatur in Gegenwart eines Zink oder Cadmium zusammen mit Silicium und Sauerstoff enthaltenden Heterogenkatalysators, und
c) Einspeisung des in der Reaktion (a) entstandenen Ketals der Formel IV in die Stufe (b) zur erneuten Herstellung des Isopropenylethers der Formel III.

2. Verfahren gemäß Anspruch 1, dadurch gekennzeichnet, daß man eine Verbindung der Formel IIa ausgewählt aus der Gruppe bestehend aus 3-Methyl-1-buten-3-ol, 3,7-Dimethyl-1-octen-3-ol, 3,7-Dimethylocta-2,6-dien-3-ol, 1-Vinylcyclohexanol, 3,7,11-Trimethyldodeca-1,6,10-trien-3-ol und 3,7,11-Trimethyldodec-1-en-3-ol mit Isopropenylmethylether umsetzt, den man durch Umsetzung von frischem und rückgeführtem Acetondimethylketal mit Propin und/oder Allen in der Gasphase bei erhöhter Temperatur in Gegenwart eines Zinksilikat-Katalysators herstellt.

3. Verfahren gemäß Anspruch 1, dadurch gekennzeichnet, daß man eine Verbindung der Formel IIb, ausgewählt aus der Gruppe bestehend aus 3-Methylbutin-3-ol, 3,7-Dimethyl-1-octin-3-ol, 3,7-Dimethylocta-6-en-1-in-3-ol, 3,7,11-Trimethyldodeca-6, 10-dien-1-in-3-ol, 3,7,11-Trimethyldodeca-6-en-1-in-3-ol und 3,7,11-Trimethyldodec-1-in-3-ol mit Isopropenylmethylether umsetzt, den man durch Umsetzung von frischem und rückgeführtem Acetondimethylketal mit Propin und/oder Allen in der Gasphase bei erhöhter Temperatur in Gegenwart eines Zinksilikat-Katalysators herstellt.

4. Verfahren gemäß Anspruch 1, dadurch gekennzeichnet, daß man die Umsetzung der Stufe (b) in Gegenwart von Katalysatoren durchführt, die eine BET-Oberfläche von 10 bis 800 m²/g aufweisen.

5. Verfahren gemäß Anspruch 1, dadurch gekennzeichnet, daß man als Katalysator für die Stufe (b) ein röntgenamorphes Zinksilikat verwendet, erhältlich durch Aufbringen eines Zinksalzes auf amorphe Kieselsäure und Formierung des Katalysators bei 50 bis 400°C.

6. Verfahren gemäß Anspruch 1, dadurch gekennzeichnet, daß man als Katalysator für die Stufe (b) ein Zinksilikat mit Hemimorphit-Struktur der Formel Zn₄Si₂O₇(OH)_{2-2y}O_{y}·xH₂O verwendet, wobei x und y für die Werte 0 bis 1 stehen.

7. Verfahren gemäß Anspruch 1, dadurch gekennzeichnet, daß man als Katalysator für die Stufe (b) ein durch Ausfällung in wässriger Lösung erhältliches im wesentlichen röntgenamorphes Zinksilikat der Formel V
ZnₐSi_{c}O_{a+2c-0,5e}(OH)ₑ·f H₂O V
verwendet, in der e die Werte 0 bis zur Summe aus 2a+4c bedeutet und das Verhältnis a/c 1 bis 3,5 und f/a 0 bis 200 beträgt.

8. Verfahren gemäß Anspruch 1, dadurch gekennzeichnet, daß man die Umsetzung der Stufe (b) bei Temperaturen von 50 bis 400°C und einem Druck von 0,1 bis 50 bar durchführt.

9. Verfahren gemäß Anspruch 1, dadurch gekennzeichnet, daß man es kontinuierlich durchführt.
